# EUROPEAN PATENT APPLICATION

(11) **EP 4 620 414 A1**
(43) Date of publication of application: **24.09.2025**
(21) Application number: 23894506.7
(22) Date of filing: 16.11.2023
(51) Int. Cl.: A61B 17/88, B25B 23/10

(54) **HOLDING INSTRUMENT AND MEDICAL DEVICE**

(30) Priority: 22.11.2022 JP 2022186148
(71) Applicant: NATIONAL UNIVERSITY CORPORATION SHIGA UNIVERSITY OF MEDICAL SCIENCE, Otsu-shi, Shiga 520-2192 (JP)
(72) Inventor: IMAI, Shinji, Otsu-shi, Shiga 520-2192 (JP)
(74) Representative: Kuttenkeuler, David
(86) International application number: PCT/JP2023/041236
(87) International publication number: WO 2024/111497

(57) **Abstract**

Provided is a retainer configured to hold a screw to be placed inside the human body, capable of holding the screw or removing the screw without damaging the tissue present around the screw. In a retainer 3 of the present invention, at a state in which a tip part 26 of a shaft 20 is placed in a hole 4, advancing a tubular body 21 toward the tip of the shaft 20 so as to expose a tip part 41 of a protruding member 22 from a groove 31 of the shaft 20 brings the tip part 41 of the protruding member 22 and the tip part 26 of the shaft 20 into firm contact with the inner surface of the hole 4 to allow the retainer to hold the screw 2, and at a state in which the screw 2 is held by the retainer 3, retracting the tubular body 21 toward the proximal end of the shaft 20 so as to release the contact of the tip part 41 of the protruding member 22 and the tip part 26 of the shaft 20 with the inner surface of the hole 4 detaches the screw 2 from the retainer 3.

## Description

### Technical Field

The present invention relates to a retainer configured to hold a screw placed inside the human body, and a medical device equipped with the retainer.

### Background Art

Traditionally, in surgeries for recurrent shoulder dislocation or other similar conditions, a screw held by a retainer is inserted into a bone graft and a recipient bone to firmly attach the bone graft to the recipient bone. Retainers, such as one equipped with multiple pinching pieces as disclosed in PTL 1, are used. The retainer of PTL 1 is composed of an inner cylinder with multiple pinching pieces extending forward in a flared manner and an outer cylinder fitted over the outer periphery of the inner cylinder. The retainer in PTL 1 can close the pinching pieces by moving the outer cylinder forward, and open the pinching pieces by moving the outer cylinder backward. The retainer in PTL 1 can also grip a screw 2 with the multiple pinching pieces or remove the screw 2 from the pinching pieces by opening and closing the pinching pieces.

### Citation List

### Patent Literature

PTL 1: JP2003-311640A

### Summary of Invention

### Technical Problem

However, the retainer of PTL 1 may damage the tissue around the screw with the multiple pinching pieces because the multiple pinching pieces are arranged outside the screw. For example, when the screw is released from the pinching pieces near blood vessels or nerves by opening the pinching pieces, the pinching pieces may damage the blood vessels or nerves.

The present invention was made in view of the current status described above, and an object of the invention is to provide a retainer configured to hold a screw to be placed inside the human body, capable of holding the screw 2 and releasing the screw without damaging the tissue present around the screw, and to provide a medical device equipped with the retainer.

### Solution to Problem

To achieve the object above, the present invention includes the subject matter described in the following Items.

### Item 1.

A retainer configured to hold a screw to be placed inside a human body, the screw having a hole that is recessed from a proximal end of the screw in an axial direction of the screw, the hole having a first polygonal shape on a cross-section,
the retainer comprising
a shaft,
a tubular body through which the shaft is inserted, and
a strip-shaped protruding member protruding from a tip of the tubular body in a longitudinal direction of the tubular body,
wherein
the shaft includes a tip part insertable into the hole of the screw with the tip part extending from the tip of the tubular body,
the tip part of the shaft has a cross-section missing a portion that constitutes a part of an outer edge of a second polygonal shape similar to the first polygonal shape, the portion being defined by a groove extending in a longitudinal direction of the shaft, the protruding member being configured to be inserted into the groove,
the groove has a bottom surface that is a tapered surface inclined toward the tip of the shaft so as to approach the part of the outer edge of the second polygonal shape,
at a state in which the tip part of the shaft is placed in the hole, advancing the tubular body toward the tip of the shaft with respect to the shaft so as to expose the tip part of the protruding member from the groove brings the tip part of the protruding member and the tip part of the shaft into firm contact with an inner surface of the hole of the screw to allow the retainer to hold the screw, and
at a state in which the screw is held by the retainer, retracting the tubular body toward a proximal end of the shaft with respect to the shaft so as to release the contact of the tip part of the protruding member and the tip part of the shaft with the inner surface of the hole of the screw detaches the screw from the retainer.

### Item 2.

The retainer according to Item 1, wherein a tip part of the tubular body is reduced in outer diameter toward the tip of the tubular body, and a proximal end part of the protruding member is joined to an outer surface of the tip part of the tubular body.

### Item 3.

The retainer according to Item 1 or 2, wherein the tip part of the protruding member protrudes opposite to the shaft.

### Item 4.

A medical device comprising the retainer of any one of Items 1 to 3 and the screw.

### Advantageous Effects of Invention

The present invention enables a screw to be held by a retainer and be removed from the retainer without damaging the tissue present around the screw.

### Brief Description of Drawings

Fig. 1 is a perspective view showing a medical device according to an embodiment of the present invention.
Fig. 2 is a perspective view showing the medical device on the tip side according to an embodiment of the present invention.
Fig. 3 is a perspective view showing a screw 2 having been detached from a retainer according to an embodiment of the present invention.
Fig. 4(A) and Fig. 4(B) show the retainer with the tubular body advanced with respect to the shaft. Fig. 4(A) is a perspective view, and Fig. 4(B) is a side view.
Fig. 5(A) and Fig. 5(B) show the retainer with the tubular body retracted with respect to the shaft. Fig. 5(A) is a perspective view, and Fig. 5(B) is a side view.
Fig. 6(A) and Fig. 6(B) show the medical device with the tubular body advanced with respect to the shaft. Fig. 6(A) is a side view showing the medical device on the tip side, and Fig. 6(B) is a cross-sectional view along line a-a of Fig. 6(A).
Fig. 7(A) and Fig. 7(B) show the medical device with the tubular body retracted with respect to the shaft. Fig. 7(A) is a side view showing the medical device on the tip side, and Fig. 7(B) is a cross-sectional view along line b-b of Fig. 7(A).

### Description of Embodiments

The following explains an embodiment of the present invention with reference to the attached drawings.

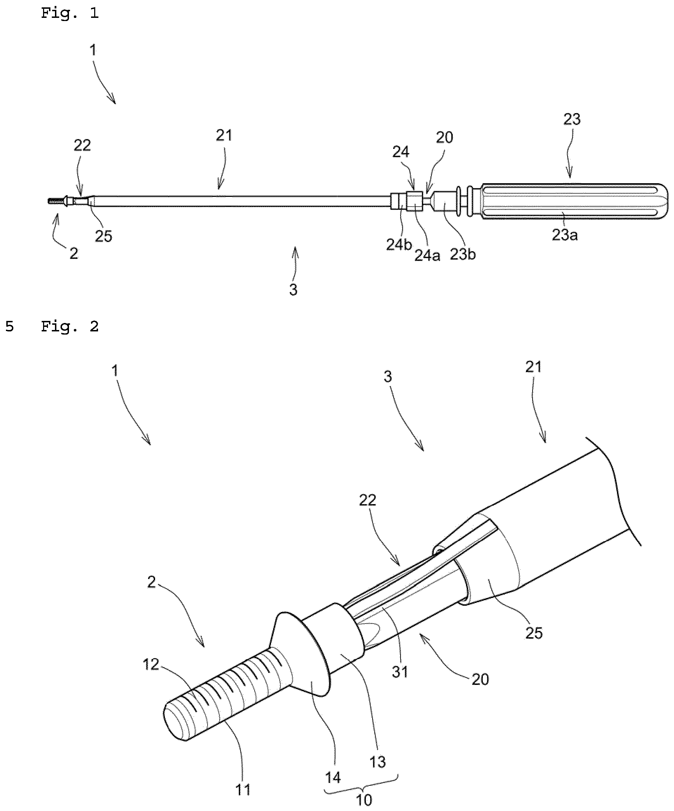

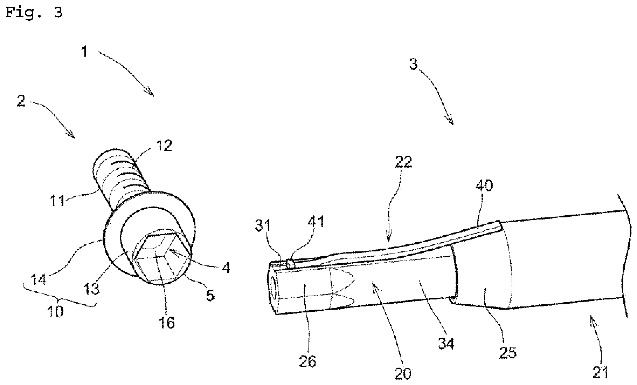

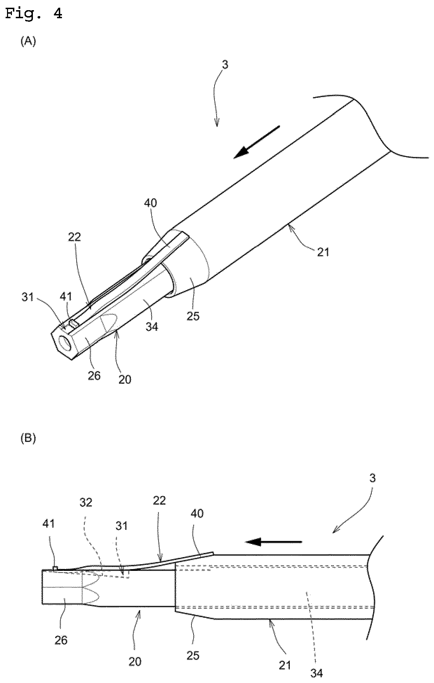

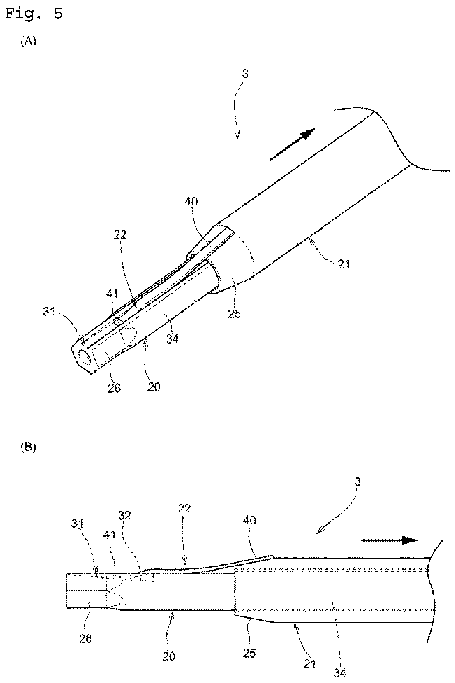

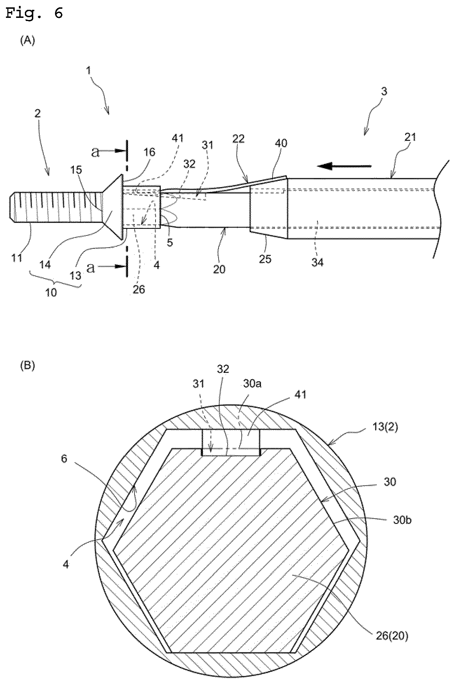

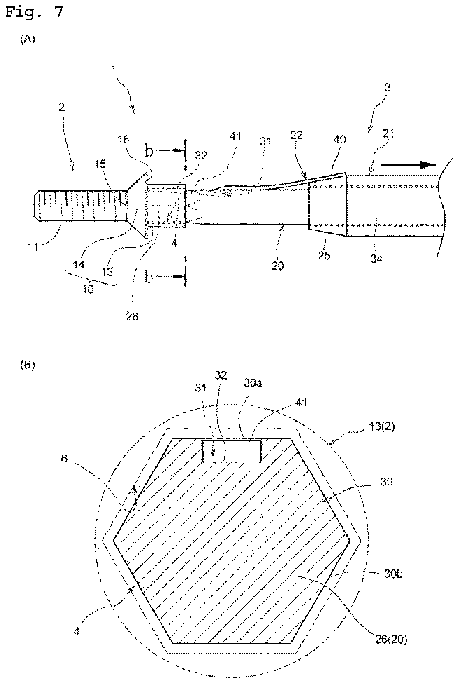

Fig. 1 is a perspective view showing a medical device 1 according to the present embodiment. Fig. 2 is a perspective view showing the medical device 1 on the tip side according to an embodiment of the present invention. Fig. 3 is a perspective view showing a screw 2 detached from a retainer 3 according to an embodiment of the present invention. Fig. 4(A) and Fig. 4(B) show the retainer 3 with the tubular body 21 advanced with respect to a shaft 20. Fig. 4(A) is a perspective view, and Fig. 4(B) is a side view. Fig. 5(A) and Fig. 5(B) show the retainer 3 with the tubular body 21 retracted with respect to the shaft 20. Fig. 5(A) is a perspective view, and Fig. 5(B) is a side view. Fig. 6(A) and Fig. 6(B) show the medical device 1 with the tubular body 21 advanced with respect to the shaft 20. Fig. 6(A) is a side view showing the medical device 1 on the tip side, and Fig. 6(B) is a cross-sectional view along line a-a of Fig. 6(A). Fig. 7(A) and Fig. 7(B) show the medical device 1 with the tubular body 21 retracted with respect to the shaft 20. Fig. 7(A) is a side view showing the medical device 1 on the tip side, and Fig. 7(B) is a cross-sectional view along line b-b of Fig. 7(A).

The medical device 1 according to an embodiment of the present invention comprises the screw 2 to be placed inside the human body and the retainer 3 configured to hold the screw 2. The medical device 1 is used, for example, in inserting the screw 2 into a bone graft and a recipient bone in surgery for recurrent shoulder dislocation in order to firmly attach the bone graft to the recipient bone.

The screw 2 is formed of metals, such as titanium, stainless steel, or cobalt alloy. As shown in Figs. 3, 6, and 7, the screw 2 has a hole 4. The hole 4 is recessed from the proximal end 5 of the screw 2 in the axial direction of the screw 2, and the cross-section of the hole 4 has a first polygonal shape 6 (in Fig. 6(B) and Fig. 7(B), reference numeral 6 indicating the outline of the first polygonal shape (the outline of the cross-section of the hole 4) is shown to specify the first polygonal shape). In the present invention, a polygon means a shape with three or more angles. In the present embodiment, the first polygonal shape 6 (the shape of the cross-section of the hole 4) is a regular hexagon; however, the first polygonal shape 6 (the shape of the cross-section of the hole 4) may be a polygon other than a regular hexagon.

In the present embodiment, the screw 2 includes a head part 10 and a screw part 11 extending from the head part 10, with a screw groove 12 formed on the outer surface of the screw part 11. The head part 10 includes an annular part 13 and a truncated cone-shaped part 14 present between the annular part 13 and the screw part 11. The truncated cone-shaped part 14 has a truncated conical shape with an outer diameter decreasing toward the screw part 11. The screw part 11 extends from an end face 15 with a small diameter (Fig. 7(A), Fig. 7(A)) of the truncated cone-shaped part 14. The annular part 13 extends from an end face 16 with a large diameter of the truncated cone-shaped part 14. The annular part 13 has an annular shape with an outer circumference, and the hole 4 is defined by the inner space of the annular part 13. The end face 16 of the truncated cone-shaped part 14 constitutes the bottom surface of the hole 4.

The present invention does not limit the structure of the screw 2 to the structure described above, and various screws with the hole 4 described above can be used as the screw 2. The present invention also does not limit the material of the screw 2 to metals, and the screw 2 can be formed from materials other than metal (e.g., resin).

As shown in Fig. 1, the retainer 3 includes the shaft 20, the tubular body 21 through which the shaft 20 is inserted, a strip-shaped protruding member 22 protruding from the tip of the tubular body 21 in the longitudinal direction of the tubular body 21, a gripping member 23 connected to the proximal end part of the shaft 20, and a rotating member 24 rotatably attached to the shaft 20 between the tubular body 21 and the gripping member 23. The shaft 20, tubular body 21, protruding member 22, and rotating member 24 are formed from metals, such as stainless steel alloy or titanium. The gripping member 23 includes a main part 23a formed of resin, such as polyethylene, and a connection part 23b formed of metal, such as stainless steel alloy or titanium, and the main part 23a is to be gripped by the user. The main part 23a has a cavity that opens on the tip side of the main part 23a (left side in Fig. 1). The proximal end of the connection part 23b is inserted into the cavity to connect the connection part 23b to the main part 23a. The connection part 23b has a cavity that opens on the tip side of the connection part 23b (left side in Fig. 1). The proximal end part of the shaft 20 is inserted into the cavity to connect the proximal end part of the shaft 20 to the gripping member 23. To allow the shaft 20 to rotate together with the gripping member 23, the cross-section of the cavity in the main part 23a, the cross-section of the cavity in the connection part 23b, and the cross-section of the proximal end part of the shaft 20 are shaped with an angular contour. The present invention does not limit the materials of the shaft 20, tubular body 21, protruding member 22, rotating member 24, and gripping member 23 to the materials described above. The shaft 20, tubular body 21, protruding member 22, rotating member 24, and gripping member 23 may be formed from materials other than those mentioned above.

The tubular body 21 has a tubular shape with a circular inner cross-section. The tubular body 21 has a tip part 25 tapered with the outer diameter decreasing toward the tip of the tubular body 21. The inner diameter of the tubular body 21 remains constant throughout the entire length of the tubular body 21. The tip part 25 of the tubular body 21 may have both the outer and inner diameters decreasing toward the tip of the tubular body 21.

The tip part 26 of the shaft 20 (Figs. 3 to 7) can be inserted into the hole 4 of the screw 2 with the tip part 26 extending from the tubular body 21. As shown in Fig. 6(B) and Fig. 7(B), the tip part 26 of the shaft 20 has "a cross-section missing a portion 31 that constitutes a part 30a of the outer edge of a second polygonal shape 30 similar to the first polygonal shape 6" (in Fig. 6(B) and Fig. 7(B), reference numeral 30 indicating the outline of the second polygonal shape is shown to specify the second polygonal shape). The second polygonal shape 30 being similar to the first polygonal shape 6 means that the second polygonal shape 30 matches the shape of the first polygonal shape 6 reduced in size while maintaining similarity. The fact that the tip part 26 of the shaft 20 has "a cross-section missing a portion 31 that constitutes a part 30a of the outer edge of a second polygonal shape 30" means that the outer edge of the tip part 26 of the shaft 20 includes a portion 30b that follows along the outer edge of the second polygonal shape 30. In the present embodiment, because the first polygonal shape 6 (the shape of the cross-section of the hole 4) is a hexagon, the second polygonal shape 30 is shown to be a regular hexagon similar to the first polygonal shape 6 as an example. However, the second polygonal shape 30 can also be a polygon other than a regular hexagon, in the same manner as the first polygonal shape 6.

The portion 31 is defined by a groove extending in the longitudinal direction of the shaft 20 (reference numeral 31 for "portion 31" is used below as a reference numeral for the groove). The bottom surface 32 of the groove 31 is a tapered surface inclined toward the tip of the shaft 20 so as to approach the part 30a of the outer edge of the second polygonal shape 30 (because the cross-section of the shaft 20 shown in Fig. 6(B) is positioned closer to the tip of the shaft 20 than the cross-section of the shaft 20 shown in Fig. 7(B), the bottom surface 32 of the groove 31 shown in Fig. 6(B) is closer to the part 30a of the outer edge in its position than the bottom surface 32 of the groove 31 shown in Fig. 7(B)).

The present invention does not particularly limit the shape of the portion 31 (groove 31) missing from the second polygonal shape 30. However, from the viewpoint of rotating the screw 2 along with the rotation of the shaft 20, it is preferable to set the shape of the portion 31 so that one or more angles of the second polygonal shape 30 are formed at the tip part 26 of the shaft 20, and it is more preferable to set the shape of the portion 31 so that all angles of the second polygonal shape 30 are formed at the tip part 26 of the shaft 20 (Fig. 6(B) and Fig. 7(B) show examples of the shaft 20 having all six angles of the second polygonal shape 30, which is a regular hexagon, formed).

The portion 34 (Figs. 3 to 7) of the shaft 20 between the tip part 26 of the shaft 20 and the proximal end part of the shaft 20 to be inserted into the cavity of the connection part 23b has a cylindrical shape insertable into the tubular body 21 over almost the entire length.

The proximal end part 40 of the protruding member 22 (Figs. 2 to 7) is joined to the tubular body 21. This joining is performed, for example, by welding. The protruding member 22 is inserted into the groove 31 of the shaft 20 with the tip part 24 of the shaft 20 extending out from the tip of the tubular body 21. In the present embodiment, the proximal end part 40 of the protruding member 22 is joined to the outer surface of the tip part 25 of the tubular body 21, so that the protruding member 22 is inclined toward the groove 31. Additionally, the tip part 41 of the protruding member 22 (Figs. 3 to 7) protrudes opposite to the shaft 20. To increase the rigidity of the protruding member 22, it is preferable to gradually increase the thickness in the portion other than the tip part 41 of the protruding member 22 toward the proximal end (toward the tubular body) (Figs. 4 to 7). However, the portion other than the tip part 41 of the protruding member 22 may have a constant thickness.

The rotating member 24 (Fig. 1) has a tubular shape with a circular inner cross-section, and a screw groove (not shown) is formed on the inner surface of the proximal end side 24a of the rotating member 24. The rotating member 24 has the shaft 20 inserted through its inside, and the screw groove on the proximal end side 24a engages with the screw groove (not shown) formed on the outer surface of the shaft 20. Additionally, an annular groove (not shown) extending in the circumferential direction of the rotating member 24 is formed on the inner surface of the distal end side 24b of the rotating member 24. With the proximal end of the tubular body 21 placed in the interior of the distal end side 24b of the rotating member 24, a protrusion (not shown) formed on the outer surface of the proximal end of the tubular body 21 is fitted into the annular groove, thereby connecting the tubular body 21 to the rotating member 24 so as not to allow the tubular body 21 to rotate together with the rotating member 24.

In the retainer 3 according to the present embodiment, rotating the rotating member 24 in the forward direction with one's finger allows the rotating member 24 and the tubular body 21 to advance toward the tip of the shaft 20 with respect to the shaft 20. Rotating the rotating member 24 in the reverse direction with one's finger also allows the rotating member 24 and the tubular body 21 to retract toward the proximal end of the shaft 20 with respect to the shaft 20. During the forward and backward movement caused by the rotation of the rotating member 24, the tubular body 21 does not rotate. During the forward movement, the protruding member 22 advances along the groove 31, and during the backward movement, the protruding member 22 retreats along the groove 31. Because the bottom surface 32 of the groove 31 is formed as a tapered surface, as described above, the advancement of the protruding member 22 allows the tip part 41 of the protruding member 22 to become exposed from the groove 31 (Fig. 6), while the retraction of the protruding member allows the tip part 41 of the protruding member 22 to become unexposed from the groove 31 (Fig. 7).

In the retainer 3 according to the present embodiment, the rotating member 24 is rotated in the forward direction with the tip part 26 of the shaft 20 placed in the hole 4 to thereby advance the tubular body 21 toward the tip of the shaft 20 with respect to the shaft 20. This allows the tip part 41 of the protruding member 22 to become exposed from the groove 31, causing the tip part 41 of the protruding member 22 and the tip part 26 of the shaft 20 to come into firm contact with the inner surface of the hole 4 of the screw 2 (Fig. 6(B)). This allows the screw 2 to stay held by the retainer 3. With the screw 2 held by the retainer 3, gripping the gripping member 23 and rotating the shaft 20 with the hand (more precisely, rotating the entire retainer 3) allow the screw 2 to rotate, and the rotation of the screw 2 causes the screw 2 to be screwed into the bone.

In the retainer 3, rotating the rotating member 24 in the reverse direction with the screw 2 held by the retainer 3 allows the tubular body 21 to retract toward the proximal end of the shaft 20 with respect to the shaft 20, thereby bringing the tip part 41 of the protruding member 22 to become unexposed from the groove 31. This releases the tip part 41 of the protruding member 22 and the tip part 26 of the shaft 20 from firm contact with the inner surface of the hole 4 of the screw 2 (Fig. 7(B)). Consequently, the screw 2 is detached from the retainer 3.

In the present embodiment, the screw 2 can be held by the retainer 3 by bringing the tip part 41 of the protruding member 22 and the tip part 26 of the shaft 20 into firm contact with the inner surface of the hole 4 of the screw 2, as described above. Releasing the contact within the hole 4 of the screw 2 allows the screw 2 to be detached from the retainer 3. In other words, in the present embodiment, it is possible to hold and remove the screw 2 without placing components of the retainer around the screw 2, unlike the prior art. Thus, the present embodiment enables the screw 2 to be held by the retainer 3 and removed from the retainer 3 without damaging the human tissue present around the screw 2.

In the present embodiment, inclination of the protruding member 22 at an angle corresponding to the slope of the outer surface of the tip part 25 allows the protruding member 22 to be inserted into the groove 31 with a minimized curvature or flexure of the protruding member 22. This allows the protruding member 22 to be strongly pressed into the hole 4 of the screw 2, bringing the tip part 41 of the protruding member 22 and the tip part 26 of the shaft 20 into firm contact with the inner surface of the hole 4. This applies an external force to the screw 2 to prevent the screw 2 from detaching from the retainer 3.

The present invention is not limited to the embodiment described above and can be modified in various ways.

For example, although the outer diameter of the tip part 25 of the tubular body 21 decreases toward the tip of the tubular body 21 in the embodiment above, the outer diameter of the tip part 25 of the tubular body 21 may be made constant. In this case, the protruding member 22 can be inserted into the groove 31 can be achieved by adjusting the shape of the protruding member 22 (e.g., by curving or bending the protruding member 22).

In the embodiment above, the tip part 41 of the protruding member 22 protrudes opposite to the shaft 20. However, it is not necessarily required for the tip part 41 of the protruding member 22 to protrude opposite to the shaft 20, as long as the tip part 41 of the protruding member 22 can be firmly in contact with the inner surface of the hole 4 of the screw 2 by advancing the protruding member 22, and as long as the contact between the tip part 41 of the protruding member 22 and the inner surface of the hole 4 can be released by retracting the protruding member 22.

Additionally, the gripping member 23 is not necessarily required and may be omitted. In this case, for example, the shaft 20 excluding the tip part 26 has a cylindrical shape insertable into the tubular body 21 over almost the entire length.

Additionally, the rotating member 24 is not necessarily required and may be omitted. In this case, for example, "advancing the tubular body 21 toward the tip of the shaft 20 with respect to the shaft 20" is achieved by pushing the tubular body 21 with the hand or pulling the shaft 20 with the hand, and "retracting the tubular body 21 toward the proximal end of the shaft 20 with respect to the shaft 20" is achieved by pulling the tubular body 21 with the hand or pushing the shaft 20 with the hand.

In the present embodiment, the inner cross-section of the tubular body 21 is circular. However, the inner cross-section of the tubular body 21 may be in a shape other than a circle. Additionally, the shape of the shaft 20 excluding the tip part 26 may be in various shapes that can be inserted into the tubular body 21.

### Description of the Reference Numerals

1 medical device
2 screw
3 retainer
5 proximal end of the screw
6 first polygonal shape
4 hole of the screw
20 shaft
21 tubular body
22 protruding member
25 tip part of the tubular body
26 tip part of the shaft
30 second polygonal shape
30a part of the outer edge of the second polygonal shape
31 portion that constitutes the part of the outer edge of the second polygonal shape, groove
32 bottom surface of the groove
40 proximal end part of the protruding member
41 tip part of the protruding member

## Claims

1. A retainer configured to hold a screw to be placed inside a human body, the screw having a hole that is recessed from a proximal end of the screw in an axial direction of the screw, the hole having a first polygonal shape on a cross-section,
the retainer comprising
a shaft,
a tubular body through which the shaft is inserted, and
a strip-shaped protruding member protruding from a tip of the tubular body in a longitudinal direction of the tubular body,
wherein
the shaft includes a tip part insertable into the hole of the screw with the tip part extending from the tip of the tubular body,
the tip part of the shaft has a cross-section missing a portion that constitutes a part of an outer edge of a second polygonal shape similar to the first polygonal shape, the portion being defined by a groove extending in a longitudinal direction of the shaft, the protruding member being configured to be inserted into the groove,
the groove has a bottom surface that is a tapered surface inclined toward the tip of the shaft so as to approach the part of the outer edge of the second polygonal shape,
at a state in which the tip part of the shaft is placed in the hole, advancing the tubular body toward the tip of the shaft with respect to the shaft so as to expose the tip part of the protruding member from the groove brings the tip part of the protruding member and the tip part of the shaft into firm contact with an inner surface of the hole of the screw to allow the retainer to hold the screw, and
at a state in which the screw is held by the retainer, retracting the tubular body toward a proximal end of the shaft with respect to the shaft so as to release the contact of the tip part of the protruding member and the tip part of the shaft with the inner surface of the hole of the screw detaches the screw from the retainer.

2. The retainer according to claim 1, wherein a tip part of the tubular body is reduced in outer diameter toward the tip of the tubular body, and a proximal end part of the protruding member is joined to an outer surface of the tip part of the tubular body.

3. The retainer according to claim 1, wherein the tip part of the protruding member protrudes opposite to the shaft.

4. A medical device comprising the retainer of any one of claims 1 to 3 and the screw.
